# EUROPÄISCHE PATENTANMELDUNG

(11) **EP 1 738 725 A1**
(43) Veröffentlichungstag der Anmeldung: **03.01.2007**
(21) Anmeldenummer: 05014226.4
(22) Anmeldetag: 30.06.2005
(51) Int. Cl.: A61F 9/007

(54) **Einrichtung für die Augenchirurgie**

(71) Anmelder: WaveLight Laser Technologie AG, 91058 Erlangen (DE)
(72) Erfinder: Donitzky, Christof, 90542 Eckental (DE); Aziret, Sinan, 90441 Nürnberg (DE)
(74) Vertreter: von Hellfeld, Axel

(57) **Zusammenfassung**

Eine Einrichtung für die Augenchirurgie umfasst eine Basiseinheit (10) mit einer Pumpanordnung (14, 16) zur Vakuumerzeugung und Flüssigkeitsabsaugung vom Auge, wobei die Basiseinheit mit einem Schlauchleitungssystem (20, 34) koppelbar ist, über welches die Pumpanordnung mit einem Evakuierungsanschluss (22) und einem Aspirationsanschluss (36) einer oder mehrerer am Auge anzubringender Komponenten (24, 38) verbindbar ist. Erfindungsgemäß umfasst die Basiseinheit gesonderte Schlauchleitungs-Koppelstellen (18, 26), mittels welcher eine mit dem Evakuierungsanschluss zu verbindende erste Schlauchleitung (20) und eine mit dem Aspirationsanschluss zu verbindende zweite Schlauchleitung (34) gleichzeitig mit der Basiseinheit koppelbar sind.

## Beschreibung

Die Erfindung betrifft eine Einrichtung für die Augenchirurgie, umfassend eine Basiseinheit mit einer Pumpanordnung zur Vakuumerzeugung und Flüssigkeitsabsaugung vom Auge, wobei die Basiseinheit mit einem Schlauchleitungssystem koppelbar ist, über welches die Pumpanordnung mit einem Evakuierungsanschluss und einem Aspirationsanschluss einer oder mehrerer am Auge anzubringender Komponenten verbindbar ist.

Eine derartige Einrichtung findet insbesondere bei der refraktiven Augenchirurgie Anwendung, bei der zur Korrektur von Sehfehlern die Hornhaut neugeformt wird. Bei modernen Techniken der refraktiven Augenchirurgie wird ein dünnes Blättchen des Hornhautepithels von den darunterliegenden Hornhautbereichen gelöst, allerdings nicht vollständig, sondern so, dass es noch an diesen darunterliegenden Hornhautbereichen hängt. Nach Wegklappen des Blättchens werden die so freigelegten Hornhautbereiche durch Ablation mittels eines Lasers, gemeinhin eines Excimer-Lasers, im Sinne einer Behebung oder zumindest Reduzierung des Sehfehlers neugeformt und anschließend das Blättchen wieder zurückgeklappt. Zur Loslösung des Blättchens von der Hornhaut sind verschiedene Techniken bekannt. Es kann ein Mikrohobel eingesetzt werden, der über die Hornhaut bewegt wird und dabei das Blättchen herausschneidet. Alternativ kann zunächst mittels eines Trepans, das ist ein Schneidrohr, das Epithel entlang eines Kreisbogens eingeschnitten werden und anschließend mittels einer Alkohollösung das Blättchen losgelöst werden. Eine ohne jede mechanische Schneideinwirkung auskommende weitere Methode verwendet einen sogenannten Femtosekundenlaser. Dieser strahlt ultrakurze Laserlichtpulse aus, die in definiert einstellbarer Tiefe der Hornhaut eine Materialtrennung bewirken und so die Loslösung des Blättchens gestatten.

Bei refraktiven Verfahren der Augenchirurgie wird üblicherweise eine Lidsperre am Auge angebracht, um die Augenlider offenzuhalten und ein Zwinkern des Patienten während der Operation zu verhindern. Dabei ist es bekannt, eine Lidsperre mit einem integrierten Absaugkanalsystem zu verwenden, das es erlaubt, durch Anschluss der Lidsperre an eine Pumpe Flüssigkeiten wie etwa Tränensekret oder Spüllösungen vom Auge abzusaugen.

Zumindest bei Verfahren mit Einsatz einer mechanischen Schneideinheit wird zudem üblicherweise eine Saugringeinheit am Auge angebracht, die zur Aufnahme und Führung der Schneideinheit dient. Die Saugringeinheit weist an ihrer dem Auge zugewandten Seite eine Ringnut auf, die bei Aufsetzen der Saugringeinheit auf den Augapfel geschlossen wird. Durch Anschluss der Saugringeinheit an eine Pumpe kann die Ringnut evakuiert werden. Hierdurch saugt sich die Saugringeinheit am Augapfel fest und sorgt für dessen Fixierung.

Bekannte Einrichtungen für die refraktive Augenchirurgie umfassen eine Basiseinheit mit einer Vakuumpumpe, die für die Erzeugung des erforderlichen Vakuums für die Fixierung des Augapfels ausgelegt ist. Die Basiseinheit weist einen einzigen Anschluss für eine Schlauchleitung auf, deren anderes Ende wahlweise mit einem Evakuierungsanschluss oder einem Aspirationsanschluss an der oder den für die Fixierung und Offenhaltung des Auges verwendeten Komponente(n) verbunden werden kann. Soll eine Saugringeinheit am Auge angebracht werden, wird die Schlauchleitung mit dem Evakuierungsanschluss verbunden. Soll Flüssigkeit vom Auge abgesaugt werden, wird dieselbe Schlauchleitung mit dem Aspirationsanschluss verbunden, der sich üblicherweise an einer Lidsperre befindet. Das Umstecken der Schlauchleitung stellt einen zusätzlichen Arbeitsschritt dar, den der operierende Arzt oder sein Assistent während der Operation durchführen muss. Dies kann störend sowohl für den Arzt als auch für den Patienten sein, denn zum einen wird der eingeübte Behandlungsablauf des Arztes unterbrochen, zum anderen erfolgt das Umstecken, während sich die Komponenten mit dem Evakuierungsanschluss und dem Aspirationsanschluss noch am Auge befinden, was sehr unangenehm und möglicherweise sogar riskant für den Patienten sein kann, wenn der Operateur hierbei nicht sanft genug vorgeht.

Aufgabe der Erfindung ist es daher, eine Einrichtung für die Augenchirurgie der eingangs bezeichneten Art bereitzustellen, die einen störungsfreien Operationsablauf gestattet und auch für den Patienten Komfortverbesserungen ermöglicht.

Zur Lösung dieser Aufgabe ist erfindungsgemäß vorgesehen, dass die Basiseinheit gesonderte Schlauchleitungs-Koppelstellen umfasst, mittels welcher eine mit dem Evakuierungsanschluss zu verbindende erste Schlauchleitung und eine mit dem Aspirationsanschluss zu verbindende zweite Schlauchleitung gleichzeitig mit der Basiseinheit koppelbar sind. Bei der erfindungsgemäßen Lösung entfällt das mühsame Umstecken einer Schlauchleitung. Der Evakuierungsanschluss und der Aspirationsanschluss können vor Beginn der Operation beide mit je einer Schlauchleitung verbunden werden, für die an der Basiseinheit je eine Koppelstelle vorhanden ist. Dadurch kann der Operateur ungestört seine Behandlung durchführen, ohne zwischendurch absetzen und eine Schlauchleitung umstecken zu müssen. Auch für den Patienten läuft die Behandlung angenehmer ab, wenn der Operateur nicht mitten in der Behandlung mehr oder weniger unsanft an einer Saugringeinheit oder einer Lidsperre hantieren muss, um eine Schlauchleitung an einer Stelle abzuziehen und an einer anderen Stelle wieder anzustecken.

Bei einer bevorzugten Weiterbildung umfasst die in der Basiseinheit untergebrachte Pumpanordnung zwei gesonderte Pumpen, denen je eine der Koppelstellen zugeordnet ist. Zur optimalen Anpassung an die unterschiedlichen Aufgaben, die von den beiden Pumpen zu erfüllen sind, nämlich Aspiration auf der einen Seite und Evakuierung auf der anderen Seite, empfiehlt es sich, Pumpen unterschiedlichen Typs zu verwenden. Vorzugsweise ist eine der Pumpen eine Rollen- oder Peristaltikpumpe, während die andere Pumpe eine Vakuumpumpe ist.

Die Aufteilung der Pumpanordnung in eine Aspirationspumpe und eine Vakuumpumpe ist vorteilhaft, weil sie eine Kontamination der Vakuumpumpe und des Evakuierungskanals durch abgesaugtes Aspirat zu vermeiden gestattet. Bei der herkömmlichen Lösung wird das Aspirat in einem Auffangbehälter aufgefangen, der in die mit der Vakuumpumpe verbundene einzige Schlauchleitung eingefügt ist. Es ist möglich, dass sich der Auffangbehälter während einer Behandlung so weit füllt, dass nachfolgend abgesaugtes Aspirat nicht mehr im Auffangbehälter zurückgehalten wird, sondern an dem Auffangbehälter vorbei in die Vakuumpumpe und die inneren Trakte der Basiseinheit gelangt. Bei der Vorsehung einer gesonderten Aspirationspumpe besteht keine Gefahr einer solchen Verunreinigung der Vakuumpumpe. Kontaminationen der Vakuumpumpe durch abgesaugtes Aspirat sind unter anderem deshalb gefährlich, weil die Vakuumpumpe oftmals in beiden Pumprichtungen betrieben wird. Um eine bei einer Augenoperation verwendete Saugringeinheit wieder vom Auge abnehmen zu können, ist es nämlich üblich, die Vakuumpumpe kurzzeitig entgegengesetzt zur Ansaugrichtung zu betreiben, um das Vakuum aufzuheben, das die Saugringeinheit am Auge festhält. Ist von einer vorangegangenen Operation eines anderen Patienten bereits Aspirat in den Trakt der Vakuumpumpe gelangt, kann nicht ausgeschlossen werden, dass Teile dieses Fremdaspirats beim Umkehrbetrieb der Vakuumpumpe in das Auge des gegenwärtigen Patienten gelangen, mit der Folge möglicher schwerer Infektionen.

Vorteilhaft an der Aufteilung der Pumpanordnung in eine Aspirationspumpe und eine Vakuumpumpe ist zudem, dass eine optimale Absaugmenge des Aspirats eingestellt werden kann. Handelsüblich erhältliche Rollen- oder Peristaltikpumpen haben sich hierzu als sehr geeignet erwiesen. Es ist nämlich zu bedenken, dass für die Aspiratabsaugung am Auge keine sehr hohe Saugkraft erforderlich ist, da bei einer zu großen abgesaugten Aspiratmenge die Bindehaut austrocknen kann und der Patient eine Zugwirkung am Auge verspüren kann, die ihn zu einer während der Operation generell unerwünschten Augenbewegung verleiten kann. Eine Rollen- oder Peristaltikpumpe kann hier eine gleichbleibend konstante, hinreichend schwache Saugkraft bieten, die solchen negativen Reaktionen vorbeugt.

Handelsüblich erhältliche Vakuumpumpen haben dagegen oftmals eine wesentlich höhere Saugkraft, die sich zwar optimal zur Fixierung des Auges eignet, jedoch für die Aspiratabsaugung vom Auge erheblich zu hoch sein kann. Insbesondere beim Einschalten der Pumpe, wenn plötzlich ein sehr starker Sog am Auge anliegt, könnte der Patient beträchtliche Irritation verspüren. Rollen- oder Peristaltikpumpen bieten hier ein deutlich sanfteres Anlaufverhalten.

Es ist darauf hinzuweisen, dass alternativ auch eine Ausgestaltung der Erfindung mit einer einzigen Pumpe vorstellbar ist, die gemeinsam dem Aspirationskanal und dem Evakuierungskanal zugeordnet ist. Hierzu können in der Basiseinheit geeignete Verzweigungsmittel enthalten sein, über die die beiden Schlauchleitungs-Koppelstellen der Basiseinheit mit einer Sogseite dieser einzigen Pumpe verbunden oder verbindbar sind. Die Verzweigungsmittel können steuerbar ausgeführt sein, so dass ein Bediener an einer Bedienseite der Basiseinheit einstellen kann, an welcher Schlauchleitungs-Koppelstelle ein Pumpsog anliegen soll oder/und wie stark dieser Pumpsog sein soll oder/und wie das Verhältnis des Pumpsogs an den beiden Schlauchleitungs-Koppelstellen zueinander sein soll. Zu diesem Zweck können die Verzweigungsmittel beispielsweise ein oder mehrere proportional steuerbare Wegeventile umfassen. Vorstellbar ist auch, dass in die Schlauchleitungen, die mit den Schlauchleitungs-Koppelstellen der Basiseinheit koppelbar sind, ein Schieber oder eine verstellbare Drossel eingebaut ist, um den Pumpsog einstellen zu können, der über die betreffende Schlauchleitung zu dem Evakuierungsanschluss bzw. dem Aspirationsanschluss übertragen wird. Die Formulierung eines selbständigen Schutzbegehrens für eine derartig ausgestaltete Schlauchleitung zur Verwendung in Verbindung mit der Basiseinheit der erfindungsgemäßen Augenchirurgieeinrichtung wird ausdrücklich vorbehalten.

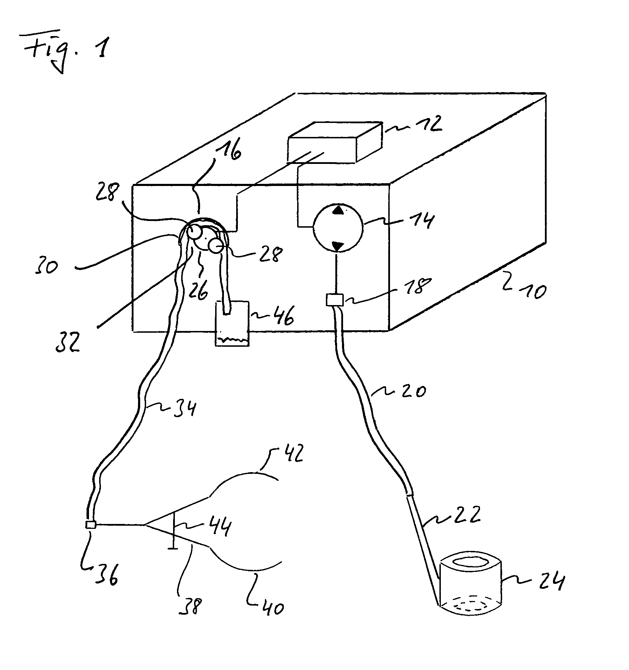

Ein Ausführungsbeispiel der Erfindung wird nun anhand der beigefügten Fig. 1 erläutert, die schematisch Komponenten einer Einrichtung zur refraktiven Augenchirurgie zeigt. Gezeigt ist eine Basiseinheit 10, die als Tischgerät ausgebildet sein kann und neben einer zentralen Steuereinheit 12 zwei Pumpen 14, 16 enthält, die zur Vakuumerzeugung und zur Flüssigkeitsabsaugung vom Auge dienen. Die Pumpe 14 ist als richtungsumsteuerbare Vakuumpumpe ausgebildet und ist in nicht näher dargestellter Weise mit einem eine erste Schlauchleitungs-Koppelstelle im Sinne der Erfindung bildenden Anschluss 18 an der Außenseite der Basiseinheit 10 verbunden. An den Anschluss 18 ist in der gezeigten Konfiguration eine erste Schlauchleitung 20 angeschlossen, die andernends an einen von einem länglichen Stutzen gebildeten Evakuierungsanschluss 22 einer nur grob schematisch angedeuteten Saugringeinheit 24 angesteckt ist. Die Saugringeinheit 24 dient zur Fixierung des Augapfels. Sie wird hierzu auf den Augapfel aufgesetzt und durch Vakuum an diesen angesaugt. Wenngleich in der Zeichnung nicht näher erkennbar, ist die Saugringeinheit 24 mit geeigneten Führungsformationen zur beispielsweise geradlinigen Führung einer Schneideinheit in Form eines Trepanrohrs oder eines Mikrohobels ausgeführt, wie es in der Fachwelt an sich bekannt ist.

Die Pumpe 16 ist im gezeigten Beispielfall als Rollenpumpe ausgebildet, welche in an sich bekannter Weise eine Mehrzahl (hier zwei) an einem rotierenden Träger 26 gehaltener Quetschrollen 28 aufweist, die zwischen sich und einem äußeren Führungsbogen 30 einen Einlegekanal 32 für eine zweite Schlauchleitung 34 begrenzen. Der Einlegekanal 32 bildet eine zweite Schlauchleitungs-Koppelstelle im Sinne der Erfindung. Die Schlauchleitung 34 ist in der gezeigten Konfiguration an einen Aspirationsanschluss 36 angeschlossen, welcher an einer ebenfalls nur grob schematisch angedeuteten, für sich jedoch bekannten Lidsperre 38 ausgebildet ist. Die Lidsperre 38 weist zwei Bügel 40, 42 auf, die am oberen bzw. unteren Augenlid einzuhaken sind und hierdurch das Auge offenhalten. Mittels einer Stellschraube 44 kann der Abstand der Bügel 40, 42 justiert werden. Die Lidsperre 38 enthält ein Kanalsystem, welches von dem Aspirationsanschluss 36 zu mehreren nicht näher dargestellten Öffnungen an den Bügeln 40, 42 verläuft. Durch diese Öffnungen kann Aspirat vom Auge abgesaugt werden. Zum Auffangen des abgesaugten Aspirats dient ein Auffangbehälter 46, in den das andere Ende der Schlauchleitung 34 lose eingehängt sein kann oder der in anderer Weise mit der Schlauchleitung 34 gekoppelt sein kann.

Die Steuereinheit 12 steuert den Betrieb der Pumpen 14, 16 nach Maßgabe von Steuerbefehlen, die ein Benutzer über nicht näher dargestellte Bedienelemente an der Basiseinheit vorgeben kann, oder/und nach Maßgabe eines in einem Speicher gespeicherten Steuerprogramms.

## Patentansprüche

1. Einrichtung für die Augenchirurgie, umfassend eine Basiseinheit (10) mit einer Pumpanordnung (14, 16) zur Vakuumerzeugung und Flüssigkeitsabsaugung vom Auge, wobei die Basiseinheit mit einem Schlauchleitungssystem (20, 34) koppelbar ist, über welches die Pumpanordnung mit einem Evakuierungsanschluss (22) und einem Aspirationsanschluss (36) einer oder mehrerer am Auge anzubringender Komponenten (24, 38) verbindbar ist,
**dadurch gekennzeichnet, dass** die Basiseinheit (10) gesonderte Schlauchleitungs-Koppelstellen (18, 26) umfasst, mittels welcher eine mit dem Evakuierungsanschluss zu verbindende erste Schlauchleitung (20) und eine mit dem Aspirationsanschluss zu verbindende zweite Schlauchleitung (34) gleichzeitig mit der Basiseinheit koppelbar sind.

2. Einrichtung nach Anspruch 1,
**dadurch gekennzeichnet, dass** die Pumpanordnung (14, 16) zwei gesonderte Pumpen umfasst, denen je eine der Koppelstellen (18, 26) zugeordnet ist.

3. Einrichtung nach Anspruch 2,
**dadurch gekennzeichnet, dass** die Pumpen (14, 16) unterschiedlichen Typs sind.

4. Einrichtung nach Anspruch 3,
**dadurch gekennzeichnet, dass** eine (16) der Pumpen eine Rollen- oder Peristaltikpumpe ist, während die andere Pumpe (14) eine Vakuumpumpe ist.

## Geänderte Patentansprüche

### Geänderte Patentansprüche gemäss Regel 86(2) EPÜ.

**1.** Einrichtung für die Augenchirurgie, umfassend eine Basiseinheit (10) mit einer Pumpanordnung (14, 16) zur Vakuumerzeugung und Flüssigkeitsabsaugung vom Auge, wobei die Basiseinheit mit einem Schlauchleitungssystem (20, 34) koppelbar ist, über welches die Pumpanordnung mit einem Evakuierungsanschluss (22) einer Saugringeinheit (24) und einem Aspirationsanschluss (36) einer Lidsperre (38) verbindbar ist,
**dadurch gekennzeichnet, dass** die Basiseinheit (10) gesonderte Schlauchleitungs-Koppelstellen (18, 26) umfasst, mittels welcher eine mit dem Evakuierungsanschluss zu verbindende erste Schlauchleitung (20) und eine mit dem Aspirationsanschluss zu verbindende zweite Schlauchleitung (34) gleichzeitig mit der Basiseinheit koppelbar sind.

**2.** Einrichtung nach Anspruch 1,
**dadurch gekennzeichnet, dass** die Pumpanordnung (14, 16) zwei gesonderte Pumpen umfasst, denen je eine der Koppelstellen (18, 26) zugeordnet ist.

**3.** Einrichtung nach Anspruch 2,
**dadurch gekennzeichnet, dass** eine (16) der Pumpen eine Rollen- oder Peristaltikpumpe ist, während die andere Pumpe (14) eine Vakuumpumpe ist.
